(19) Europäisches Patentamt
European Patent Office
Office européen des brevets

(11) Publication number: **0 137 627 B1**

(12) **EUROPEAN PATENT SPECIFICATION**

(45) Date of publication of patent specification: **22.01.92** (51) Int. Cl.$^5$: **A61K 47/00**

(21) Application number: **84305443.8**

(22) Date of filing: **09.08.84**

(54) **Pour-on formulation for the control of parasites.**

(30) Priority: **12.08.83 AU 816/83**

(43) Date of publication of application:
**17.04.85 Bulletin 85/16**

(45) Publication of the grant of the patent:
**22.01.92 Bulletin 92/04**

(84) Designated Contracting States:
**AT BE CH DE FR GB IT LI LU NL SE**

(56) References cited:
**GB-A- 2 088 212**
**GB-A- 2 110 090**
**GB-A- 2 110 091**

(73) Proprietor: **PITMAN-MOORE AUSTRALIA LIMITED**
**71, Epping Road**
**North Ryde New South Wales 2113(AU)**

(72) Inventor: **Evans, David Alwyn Charles**
**9 Ballarat Street**
**Collingwood Victoria, 3066(AU)**
Inventor: **Hamilton, Robert John**
**38 Steele Street**
**Moonee Ponds Victoria, 3039(AU)**
Inventor: **Wilshire, Colin**
**17 Harry Street**
**Doncaster East Victoria, 3109(AU)**

(74) Representative: **Bannerman, David Gardner et al**
**Withers & Rogers 9 The Parade**
**Leamington Spa Warwickshire CV32 5DG(GB)**

EP 0 137 627 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to compositions suitable for the topical application of chemicals to animals to control parasites. In particular it relates to compositions comprising systemically active chemicals for the control of parasites.

Oral and parenteral administration of parasiticides for the control of endoparasites are well known in the art and usually take the form of drenching or injecting animals with suitable formulations of the parasiticides. However, because of their inherent advantages, especially in ease of administration, recently much effort has been directed to the development of pour-on formulations. That is, formulations which when topically applied by pouring onto or spotting onto the surface of the animal, typically along the backline or spinal region, provide effective control of endoparasites. The pour-on method of administration has inherent advantages over oral and parenteral administration including, for example, ease of administration, removal of the need to prevent the animal from moving during administration, and easing of the requirement to maintain sterile formulations. Moreover, highly trained personnel are not required for topical administration of parasiticide formulations.

One parasiticide which is particularly favoured for the control of endoparasites in animals is tetramisole (d,1-2,3,5,6-tetrahydro-6-phenylimidazo-[2,3-b]thiazole) and its laevo-rotatory optical isomer levamisole. Levamisole is now the more widely used product as it is twice as active as tetramisole and has twice the therapeutic ratio. In practice the inorganic salts of levamisole such as, for example, levamisole hydrochloride and levamisole dihydrogen phosphate, formulated in highly acidic aqueous solutions, have been used for drenching and injection. Tetramisole and levamisole have been described in US Patent Nos 3 274 209 and 3 463 786 respectively.

In view of the efficacy of levamisole in the control of endoparasites much of the work directed to the development of a pour-on formulation for the control of endoparasites has investigated the use of levamisole. Moreover, as the inorganic salts of levamisole which are used in drench and injectable compositions show negligible skin penetration, the investigations have concentrated on the use of levamisole per se, that is levamisole base, and not on the use of inorganic salts of levamisole.

Pour-on compositions comprising tetramisole, levamisole and the acid-addition salts thereof have been developed which are effective in transporting the endoparasiticide through the skin of cattle. For example, Australian Patent Nos 489 412 and 490 027 disclose formulations in which the carriers include solvents such as spindle oil and isopropanol, aromatic hydrocarbons (eg xylene and benzene), ketones (eg cyclohexanone), and various alcohols. However, the carriers disclosed which are most effective in transporting the endoparasiticde through the skin may also produce pain and severe tissue reactions such as swelling, dryness and cracking of the skin. Moreover, such compositions have been found to be generally ineffective as endoparasiticides when applied to sheep and to cause pain and severe skin reactions including severe damage to the hides and skin of the animals.

Australian Patent Application No 71 322/81 and its equivalent United States Patent 4 278 684 disclose the use of aliphatic carboxylic acids of pKa in the range of from 3 to 6 as skin penetration promoters to improve the efficacy of pour-on compositions comprising tetramisole or levamisole and a di(lower alkyl) dicarboxylic acid ester. The applicants have confirmed that the use of aliphatic carboxylic acids of pKa in the range of from 3 to 6 does enhance the uptake of the endoparasiticide in sheep but no improvement in uptake was noted in cattle. However, the use of alkanoic acids in pour-on compositions to promote the uptake of the endoparasiticide was found to increase the level of adverse skin reactions in sheep, and especially in Merino and Merino-cross sheep which appear to be particularly susceptable to adverse skin reaction. Moreover, it was further found that the use of alkanoic acids in pour-on formulations comprising a hydroxylic solvent such as an alcohol (eg the compositions exemplified in United States Patent No 4 278 684) led to rapid decrease in the efficacy of the compositions on storage through degradation of the endoparasiticides levamisole and tetramisole.

Australian Patent Application No 81782/82 discloses a pour-on composition comprising tetramisole or levamisole in a carrier comprising at least 50% by weight of an alcohol ethoxylate. The application teaches that the use of an alcohol ethoxylate as a carrier, and especially at least 80% by weight of 2-(2-butoxyethoxy)ethanol as carrier, decreases the incidence of skin irritation and the specification exemplifies the use of such formulations in the treatment of cattle. The applicants have confirmed that such formulations are suitable for use in cattle. However, the applicants have found that such formulations are not suitable for use in sheep as they fail to give anthelmintically effective blood levels of the endoparasiticide, presumably through unsatisfactory transfer of the endoparasiticide through wool or wool grease cover, and their use results in unacceptable adverse skin reaction.

Australian Patent Application No 90 937/82 discloses the use of a mixture of a penetrating solvent and a

bland solvent as a carrier to reduce or eliminate the incidence of adverse skin reactions in pour-on compositions comprising tetramisole or levamisole. In practice it has been found that such carriers, comprising an alcohol alkoxylate and a dialkyl ester of a dicarboxylic acid, dicarboxylate ester of a dihydric alcohol or a carboxylate ester of an alcohol alkoxylate, provide excellent pour-on compositions for the effective anthelmintic treatment of cattle and essentially overcome the problem of adverse skin reaction in sheep. However, under field conditions the compositions did not provide consistent anthelmintically effective blood levels of the endoparasiticide in all of the breeds of sheep tested.

While the prior-art discloses formulations which have provided progressively increasing endoparasiticidal efficacy and decreasing adverse skin reaction there remains a need in the art for an endoparasiticidal pour-on formulation with improved efficacy and decreased adverse skin reaction. In particular, there remains a need for an endoparasiticidally effective pour-on formulation which may be applied to sensitive animals such as sheep without causing adverse skin reaction.

New endoparasiticidal pour-on formulations have now been found which, surprisingly, combine the features of improved efficacy, freedom from unacceptable skin reactions even when applied to sensitive breeds of animals, and long shelf life even when they incorporate levamisole or tetramisole which are relatively chemically sensitive and subject to decomposition.

Accordingly, the invention provides a composition for topical application to animals to control endoparasites which composition comprises an endoparasiticide and a carrier comprising at least one saturated aliphatic carboxylate ester of a mono alkyl ether of a mono- or poly- alkylene glycol as defined in the claims.

Endoparasiticides which may be used in the compositions of the present invention include: anthelmintics such as tetramisole, levamisole and avermectins including ivermectin; and flukicides such as triclabendazole [6-chloro-5-(2,3-dichlorophenoxy)-2-methylthiobenzimidazole] and rafoxanide { N-[3-chloro-4-(4-chlorophenoxy)phenyl]-2-hydroxy-3,5-diiodobenzamide } . Preferred endoparasiticides for use in the compositions of the present invention include tetramisole and levamisole, levamisole being more preferred because of its higher anthelmintic activity and therapeutic ratio.

Suitable esters for use as carriers in the compositions of the invention include $C_2$ to $C_7$ saturated aliphatic carboxylate esters of mono-($C_1$ to $C_6$ alkyl) ethers of ethylene glycols and propylene glycols.

Preferred esters for use as carriers in the compositions of the invention include compounds of formula I

$$R^1-O-(CH_2-\overset{\overset{\large R^2}{|}}{C}HO)_n-COR^3 \qquad \text{I}$$

wherein

- $R^1$ is selected from $C_1$ to $C_6$ alkyl;
- $R^2$ is selected from hydrogen and methyl;
- $R^3$ is selected from $C_1$ to $C_6$ alkyl; and
- n is an integer selected from 1 to 3.

More preferred esters include those compounds of formula I in which:

- $R^1$ is selected from $C_2$ to $C_6$ alkyl;
- $R^2$ is selected from hydrogen and methyl;
- $R^3$ is selected from $C_1$ to $C_4$ alkyl; and
- n is selected from 1 and 2.

Examples of the more preferred esters include the $C_2$ to $C_4$ carboxylic acid esters of 2-(n-butoxy)-ethanol, 2-[2-(n-butoxy)ethoxy]ethanol, 1-(n-butoxy)propan-2-ol, 2-(n-propoxy)ethanol, 2-[2-(n-propoxy)-ethoxy]ethanol, 1-(n-propoxy)propan-2-ol, 2-ethoxyethanol, 2-(2-ethoxyethoxy)ethanol, and 1-ethoxypropan-2-ol.

Specific examples of the more preferred esters include 2-(n-butoxy)ethyl acetate, 1-ethoxyprop-2-yl acetate, 1-(n-propoxy)prop-2-yl acetate, 2-(n-butoxy)ethyl butyrate, 1-(n-butoxy)prop-2-yl acetate and 2-[2-(n-butoxy)ethoxy]ethyl acetate.

The carriers used in the compositions of the present invention may comprise, in addition to at least one saturated aliphatic carboxylate ester of a mono alkyl alkylene glycol ether, one or more organic solvents. Suitable additional organic solvents include: dialkyl esters of dicarboxylic acids such as, for example, di($C_1$ to $C_6$ alkyl)esters of $C_2$ to $C_6$ dicarboxylic acids; di(carboxyl)esters of alkylene glycols such as, for example, di($C_2$ to $C_6$ carboxyl) esters of alkylene glycols such as, for example, di($C_2$ to $C_6$ carboxyl) esters of ethylene glycol, diethylene glycol, triethylene glycol, propylene glycol and butan-1,4-diol; tri($C_2$ to $C_6$

carboxyl) esters of glycerol; and mono- alkyl ethers of alkylene glycols such as, for example, the products of reaction of one mole of a $C_1$ to $C_6$ alcohol with from one to three moles of an alkylene oxide selected from ethylene oxide, propylene oxide and mixtures thereof. However, preferably the optional, additional organic solvent is a non-hydroxylic solvent.

Preferred optional, additional organic solvents include the di($C_2$ to $C_6$ carboxyl) esters of ethylene glycol, diethylene glycol, triethylene glycol, propylene glycol and butan-1,4-diol. Specific examples of the preferred optional, additional organic solvents include ethylene glycol diacetate, diethylene glycol diacetate and butan-1,4-diol diacetate.

When an optional, additional organic solvent is employed in the compositions of the present invention preferably the weight ratio of the ester of the composition of the present invention to the optional, additional carrier is in the range of from 9:1 to 1:2.

The compositions of the present invention optionally may also comprise one or more aliphatic carboxylic acids. In general it has been found that the compositions of the present invention do not require the addition of carboxylic acids to promote uptake of the endoparasiticide. However, in some carriers comprising tetramisole or levamisole as endoparasiticide the presence of an aliphatic carboxylic acid may promote the uptake of the endoparasiticide. When used in tetramisole or levamisole compositions of the present invention preferably the molar ratio of the aliphatic carboxylic acid to the tetramisole or levamisole does not exceed 1.1 and more preferably it is in the range of 0.5 to 1.0. Preferred aliphatic carboxylic acids for use in tetramisole or levamisole containing compositions of the present invention are the $C_1$ to $C_8$ alkanoic acids, more preferably $C_1$ to $C_4$ alkanoic acids, and especially acetic acid and propionic acid.

In compositions of the present invention comprising tetramisole or levamisole as endoparasiticide and a hydroxylic solvent as an additional organic solvent, preferably aliphatic carboxylic acids are not added in order to attempt to promote the uptake of the endoparasitcde. Surprisingly, it has been found that the endoparasiticides tetramisole and levamisole undergo relatively rapid degradation in the presence of hydroxylic solvents and aliphatic carboxylic acids and as a result such compositions have an unsatisfactory shelf life. Therefore, as hereinbefore indicated, preferably any optional, additional organic solvent incorporated into the compositions of the present invention is a non-hydroxylic solvent.

The compositions of the present invention may optionally contain additives to stabilize, preserve, colour or otherwise improve the storage properties and/or ease of application of the compositions. Examples of stabilizers which may find application in the compositions of the present invention are antioxidants such as, for example, phenol antioxidants. Included among the preferred antioxidants is the phenol antioxidant 2,6-di-(t-butyl)-4-methylphenol. When used in the compositions of the present invention the antioxidant or mixture of antioxidants is preferably used at a concentration in the range of from 0.01 to 1.0 percent w/v, more preferably 0.05 to 0.1 percent w/v of the final composition.

It will be evident to those skilled in the art that in certain circumstances it may be desirable for the compositions of the invention to contain a colouring agent or dye. For example, the presence of a colouring agent in the compositions of the invention may provide a quick, effective way for identifying the compositions per se. Moreover, animals treated by topical application of the compositions may be readily identified thereby allowing unnecessary and undesirable duplicate treatment to be avoided. When used in the compositions of the present invention preferably the colouring agent or dye is non-fast and/or watersoluble so that the colouring agent or dye will fade or be washed away within a suitable period without deleterious effect to the skin or wool. Colouring agents or dyes which have been found to be useful in the compositions of the present invention include edicol erythrosine and rhodamine B. When used in the compositions of the present invention preferably the colouring agent or dye is used at a concentration in the range of from 0.001 to 0.05 percent w/v of the final composition.

Further optional additives which will be evident to those skilled in the art include, for example, bitter tasting non-toxic agents to deter the animals from licking the compositions off their hide or skin or the hides or skins of other animals.

The compositions of the present invention may include, in addition to one of more endoparasiticides, one or more agents which, when topically applied to animals, are effective in treating diseases of animals or generally improving the health and/or well being of animals. Particularly useful agents which may be incorporated into the compositions of the invention are ectoparasiticides which are useful in the control or eradication of animal ectoparasites. Examples of preferred ectoparasiticides which may be used in the compositions of the invention are synthetic pyrethroids such as, for example, permethrin, cypermethrin, deltamethrin, phenothrin, cyphenothrin, flumethrin, cyfluthrin and cyhalothrin.

When a synthetic pyrethroid is used as an ectoparasiticide in the compositions of the present invention the concentration of the pyrethroid in the formulation will depend to a large extent on the pyrethroid used, the ectoparasite to be controlled and the animal to be treated. The specific concentration may be readily

determined by those skilled in the art so that topical application of the quantity of the composition required to give effective control of endoparasites will also give effective control of ectoparasites. Typically, the concentration of the present invention will be in the range of from 0.2 to 5.0 percent w/v of the final composition.

The invention also provides in a further embodiment a process for the control or eradication of endoparasites in an animal which process comprises topically applying to said animal an endoparasiticidally effective amount of a composition as hereinbefore defined.

In the method of treatment according to the invention, the compositions of the invention are topically applied to the animal generally by pouring onto or spotting onto the surface of the animal along the backline or spinal region. The compositions may also be applied by brushing or rolling onto the surface of the animal but, for convenience, the compositions are preferably applied by simply pouring onto the back of the animal.

The compositions of the present invention may be used in the treatment of endoparasite infestations in a range of animals and especially warm-blooded animals including mammals. The compositions may be used in the treatment of domestic or farm animals such as, for example, cattle, sheep, pigs, horses, goats, dogs and cats, and find particular utility in the treatment of endoparasite infestations in cattle and sheep.

As hereinbefore indicated, endoparasiticides which may be used in the compositions of the present invention include: anthelmintics such as tetramisole, levamisole and avermectins including ivermectin; and flukicides such as triclabendazole and rafoxanide. The concentration of the endoparasiticide used in the compositions of the invention will depend to a large extent on the specific endoparasiticide to be used, the animal to be treated, and the amount of composition to be applied. The specific concentration may be readily determined by those skilled in the art so that when the selected quantity of the composition of the endoparasiticide is topically applied to an animal of known bodyweight, then the endoparasiticide will be applied in an amount sufficient to effectively control the infestation of endoparasites in the animal. Typically, in the compositions of the present invention, the concentrations of the endoparasiticides would be in the following ranges: tetramisole or levamisole from 1 to 30% w/v; ivermectin from 0.01 to 2.0% w/v; triclabendazole from 0.5 to 15.0% w/v; and rafoxanide from 0.5 to 15.0% w/v. Typically the amount of composition of the present invention applied to the animal to be treated is sufficient to give dose rates of the endoparasiticides in the following dose ranges: levamisole from 10 to 25 mg/kg; tetramisole from 15 to 50 mg/kg; ivermectin from 0.2 to 1.0 mg/kg; triclabendazole from 2 to 10 mg/kg; and rafoxanide from 10 to 20 mg/kg.

As hereinbefore indicated the preferred endoparasiticide for use in the compositions of the present invention is levamisole. Levamisole is a particularly effective endoparasiticide against a range of gastro intestinal and pulmonary nematodes such as, for example, Haemonchus spp., Ostertagia spp., Trichostrongylus spp., Chabertia spp. and Dictyocaulus spp. Preferably levamisole is applied at a dose rate of approximately 20 mg/kg of animal bodyweight. Therefore, a composition containing, for example, 10% w/v of levamisole would preferably be applied at a rate of 0.2 ml/kg whereas a composition containing 20% w/v of levamisole would preferably be applied at a rate of 0.1 ml/kg.

The compositions of the present invention provide a significant advance over prior-art compositions as they combine the features of improved efficacy (readily demonstrated by the higher endoparasiticide blood levels achieved by the compositions of the present invention), freedom from unacceptable adverse skin reaction, and long shelf life. The higher endoparasiticide blood levels obtained by topical application of the compositions of the present invention is both unexpected and significant. For example, in direct comparison trials using compositions of the present invention and compositions of Australian Patent Application No 90937/82, the compositions of the present invention gave levamisole blood levels of the order of 2 to 3 times higher in cattle and 4 to 8 times higher in sheep. Moreover, no unacceptable adverse skin reaction was noted either in the sheep or the cattle.

Another surprising feature of the compositions of the present invention is their ability to provide effective treatment of endoparasite infestations in sheep when applied topically to sheep having long wool growth. When applied topically to long-woolled sheep the compositions of the invention provide effective endoparasite control whether applied at the top of the wool or at skin level. This feature of the compositions of the present invention is completely unexpected as it is known in the art that complex organic molecules can bind strongly to wool and it would be expected from the prior art that a composition applied at the top of the wool would provide little or no percutaneous absorption of the endoparasiticide.

The compositions of the present invention may be prepared by dissolving the endoparasiticide, any additional topically effective agent for treating diseases of animals or generally improving the health and/or well being of animals, and any additive to improve the storage properties and/or ease of application of the compositions, in a carrier as hereinbefore defined. Accordingly in a further aspect the invention provides a

process for the preparation of a composition for topical application to animals to control endoparasites which process comprises dissolving said endoparasiticide in a carrier comprising at least one saturated aliphatic carboxylate ester of a mono-alkyl ether of a mono- or poly-alkylene glycol.

Typically, the compositions of the present invention are prepared by dissolving the endoparasiticide in the carrier at ambient temperature. However, lower or higher temperatures may be utilized if required, temperatures in the range of from 0 to 100 °C being preferred and temperatures in the range of from 15 to 40 °C being more preferred. The mixtures of ingredients may be agitated by, for example, shaking or stirring in order to aid dissolution of the endoparasiticide in the carrier.

The invention is now illustrated by but not limited to the following examples in which all parts and percentages are by weight unless otherwise specified.

Examples 1 to 20

These Examples illustrate the preparation of compositions of the present invention.

The compositions were prepared by mixing, at room temperature, the components in the proportions indicated in Table 1 and stirring the mixture until a homogeneous solution was obtained.

TABLE 1

| Component | Component Concentration (parts by weight) Example No 1 | 2 | 3 | 4 | 5 | 6 | 7 | 8 |
|---|---|---|---|---|---|---|---|---|
| L | 9.80 | 9.80 | 9.71 | 19.05 | 9.80 | 9.80 | 10.31 | 9.80 |
| R | - | - | - | - | - | - | - | - |
| I | - | - | - | - | - | - | - | - |
| T | - | - | - | - | - | - | - | - |
| C | - | - | - | - | - | - | - | - |
| BEA | 57.01 | 53.45 | - | 37.93 | 56.96 | 56.96 | - | - |
| BEEA | - | - | 90.28 | - | - | - | - | - |
| EPA | - | - | - | - | - | - | 89.63 | 56.97 |
| PPA | - | - | - | - | - | - | - | - |
| BEB | - | - | - | - | - | - | - | - |
| BPA | - | - | - | - | - | - | - | - |
| EGDA | 33.18 | 33.18 | - | 42.97 | 33.18 | 33.18 | - | 33.17 |
| DEGDA | - | - | - | - | - | - | - | - |
| BGDA | - | - | - | - | - | - | - | - |
| EE | 0.01 | 0.01 | 0.01 | - | 0.01 | - | - | 0.01 |
| RB | - | - | - | - | - | 0.01 | 0.01 | - |
| PA | - | 3.56 | - | - | - | - | - | - |
| BP | - | - | - | 0.05 | 0.05 | 0.05 | 0.05 | 0.05 |
| DENSITY ($g/cm^3$) | 1.02 | - | 1.03 | 1.05 | 1.02 | 1.02 | 0.97 | 1.02 |

TABLE 1 - continued

| Com-ponent | Component Concentration (parts by weight) Example No 9 | 10 | 11 | 12 | 13 | 14 | 15 | 16 |
|---|---|---|---|---|---|---|---|---|
| L | 9.80 | 9.90 | 9.80 | 4.85 | 9.71 | 9.52 | 9.80 | - |
| R | - | - | - | - | - | - | - | 8.62 |
| I | - | - | - | - | - | - | - | - |
| T | - | - | - | - | - | - | - | - |
| C | - | - | - | - | - | - | 0.98 | - |
| BEA | - | - | - | 62.24 | - | 59.04 | 55.98 | 62.21 |
| BEEA | - | - | - | - | - | - | - | - |
| EPA | - | - | - | - | 60.28 | - | - | - |
| PPA | 57.01 | - | - | - | - | - | - | - |
| BEB | - | 90.09 | - | - | - | - | - | - |
| BPA | - | - | 90.14 | - | - | - | - | - |
| EGDA | 33.18 | - | - | 32.85 | - | - | 33.18 | 29.17 |
| DEGDA | - | - | - | - | - | 31.43 | - | - |
| BGDA | - | - | - | - | 30.00 | - | - | - |
| EE | 0.01 | 0.01 | 0.01 | 0.01 | 0.01 | 0.01 | 0.01 | |
| RB | - | - | - | - | - | - | - | |
| PA | - | - | - | - | - | - | - | |
| BP | - | - | 0.05 | 0.05 | - | - | 0.05 | |
| DENSITY ($g/cm^3$) | 1.02 | 1.01 | 1.02 | 1.03 | 1.03 | 1.05 | - | 1.12 |

TABLE 1 - continued

| Component | Component Concentration (parts by weight) Example No 17 | 18 | 19 | 20 |
|---|---|---|---|---|
| L | - | - | - | - |
| R | 5.21 | - | - | - |
| I | - | 0.50 | - | 0.50 |
| T | - | - | 5.21 | - |
| C | - | - | - | - |
| BEA | - | - | - | 55.70 |
| BEEA | - | - | - | - |
| EPA | 94.79 | 99.5 | 94.79 | - |
| PPA | - | - | - | - |
| BEB | - | - | - | - |
| BPA | - | - | - | - |
| EGDA | - | - | - | 43.80 |
| DEGDA | - | - | - | - |
| BGDA | - | - | - | - |
| EE | - | - | - | - |
| RB | - | - | - | - |
| PA | - | - | - | - |
| BP | - | - | - | - |
| DENSITY ($g/cm^3$) | 0.96 | 0.96 | 0.96 | 1.03 |

Code for TABLE 1

| | | |
|---|---|---|
| L | - | Levamisole |
| R | - | Rafoxanide |
| I | - | Ivermectin |
| T | - | Triclabendazole |
| C | - | Cyhalothrin |
| | | |
| BEA | - | 2-(n-Butoxy)ethyl acetate |
| BEEA | - | 2-[2-(n-Butoxy)ethoxy]ethyl acetate |
| EPA | - | 1-Ethoxyprop-2-yl acetate |
| PPA | - | 1-(n-Propoxy)prop-2-yl acetate |
| BEB | - | 2-(n-Butoxy)ethyl butyrate |
| BPA | - | 1-(n-Butoxy)prop-2-yl acetate |
| | | |
| EGDA | - | Ethylene glycol diacetate |
| DEGDA | - | Diethylene glycol diacetate |
| BGDA | - | Butan-1,4-diol diacetate |
| | | |
| EE | - | Edicol erythrosine |
| RB | - | Rhodamine B |
| | | |
| PA | - | Propionic Acid |
| | | |
| BP | - | 2,6-Di-(t-Butyl)-4-methylphenol |

Examples 21 to 35

These Examples illustrate the efficacy of compositions of the present invention in producing surprisingly high and anthelmintically effective blood levels of levamisole in sheep.

The test animals were treated with a composition of the invention by pour-on application along the spine. Details of the animals treated, dose rate and composition of the invention used are given in Table 2.

TABLE 2

| Example No | Test Animal | Dose Rate (mg/kg) | Test Composition (Example No) |
|---|---|---|---|
| 21 | Merino sheep | 20 | 1 |
| 22 | Merino sheep | 20 | 2 |
| 23 | Merino sheep | 10 | 2 |
| 24 | Merino sheep (freshly shorn) | 20 | 3 |
| 25 | Merino sheep (freshly shorn) | 20 | 8 |
| 26 | Merino sheep (freshly shorn) | 20 | 9 |
| 27 | Merino sheep (freshly shorn) | 20 | 10 |
| 28 | Merino sheep (freshly shorn) | 20 | 11 |
| 29 | Merino sheep (long woolled; 8-10 cm) | 20 | 12[a] |
| 30 | Merino sheep (long woolled; 8-10 cm) | 20 | 12[b] |
| 31 | Merino sheep (long woolled; 8-10 cm) | 20 | 12[c] |
| 32 | Merino sheep (long woolled; 8-10 cm) | 20 | 1[a] |
| 33 | Merino sheep (long woolled; 8-10 cm) | 20 | 1[b] |
| 34 | Merino sheep (long woolled; 8-10 cm) | 20 | 1[c] |
| 35 | Merino sheep (freshly shorn) | 20 | 13 |

Code for TABLE 2:

a - Composition applied as a stripe along the back-line at the top of the fleece.

b - Composition applied by spotting at the top of the fleece.

c - Composition applied by spotting at skin level.

Blood samples were drawn from the animal at regular intervals and the resulting serum was analyzed for levamisole by a procedure involving clean-up on "Bond Elut" $C_{18}$ columns ("Bond Elut" is a registered trade mark) followed by a final determination by high performance liquid chromatography.

The results are shown in Tables 3 to 17 and illustrate that blood levamisole levels of 0.3 $\mu$ g/cm$^3$ and higher are rapidly reached and maintained over a period of seven hours. A blood levamisole level of 0.3 $\mu$ g/cm$^3$ or above is known in the art to give effective control of helminth endoparasites. No unacceptable adverse skin reactions were noted during or after the application of any of the compositions.

The results shown in Tables 11 to 16 illustrate a further unique feature of the compositions of the invention. That is, the suitability of the compositions of the present invention for the effective treatment of endoparasite infestations by topical application to either shorn or long-woolled sheep. This feature of the compositions of the invention is completely unexpected as it is known in the art that chemicals can bind strongly to wool ["Topical Drug Delivery to Cattle and Sheep", I H Pitman and S J Rostas, J. Pharm. Sci., 70 (11), 1181-1194 (1981)] and it would be expected from the prior art that a levamisole composition topically applied to long woolled sheep would result in little or no percutaneous absorption of levamisole.

TABLE 3 - Example 21

| Animal No | Levamisole conc. ( μ g/cm³) in Serum | | | | |
|---|---|---|---|---|---|
| | Hours: 1 | 2 | 3 | 5 | 7 |
| 1 | 1.59 | 2.17 | 3.02 | 2.05 | 1.91 |
| 2 | 0.38 | 0.60 | 0.85 | 1.00 | 1.15 |
| 3 | 0.28 | 0.49 | 0.68 | 0.88 | 0.85 |
| 4 | 0.38 | 0.74 | 1.29 | 1.11 | 0.92 |
| 5 | 0.57 | 1.51 | 2.14 | 2.18 | 1.56 |
| 6 | 1.09 | 2.08 | 2.62 | 2.57 | 1.52 |
| 7 | 0.43 | 0.92 | 1.35 | 1.57 | 1.37 |
| 8 | 0.30 | 0.39 | 0.63 | 0.41 | 0.41 |
| 9 | 0.21 | 0.30 | 0.52 | 0.63 | 0.62 |
| Mean | 0.58 | 1.02 | 1.46 | 1.37 | 1.15 |

TABLE 4 - Example 22

| Animal No | Levamisole conc. ( μ g/cm$^3$) in Serum | | | | |
|---|---|---|---|---|---|
| | Hours: 1 | 2 | 3 | 5 | 7 |
| 1 | 0.73 | 1.33 | 1.63 | 1.38 | 0.96 |
| 2 | 2.20 | 2.36 | 2.17 | 1.35 | 0.94 |
| 3 | 0.41 | 0.49 | 0.60 | 0.53 | 0.51 |
| 4 | 0.95 | 1.51 | 1.77 | 1.77 | 1.08 |
| 5 | 0.77 | 1.51 | 1.77 | 1.63 | 1.23 |
| 6 | 0.94 | 1.76 | 1.97 | 1.50 | 1.44 |
| 7 | 1.31 | 2.25 | 1.89 | 1.33 | 0.77 |
| 8 | 0.85 | 1.39 | 1.79 | 1.77 | 1.56 |
| 9 | 0.91 | 1.88 | 2.48 | 1.78 | 1.57 |
| 10 | 1.77 | 2.33 | 2.54 | 1.63 | 0.92 |
| Mean | 1.08 | 1.68 | 1.86 | 1.45 | 1.10 |

TABLE 5 - Example 23

| Animal No | Levamisole conc. ( μ g/cm$^3$) in Serum | | | | |
|---|---|---|---|---|---|
| | Hours: 1 | 2 | 3 | 5 | 7 |
| 1 | 0.34 | 0.73 | 0.78 | 0.67 | 0.50 |
| 2 | 0.41 | 0.78 | 0.96 | 0.65 | 0.31 |
| 3 | 0.78 | 1.29 | 1.49 | 1.61 | 1.10 |
| Mean | 0.51 | 0.93 | 1.08 | 0.98 | 0.64 |

TABLE 6 - Example 24

| Animal No | Levamisole conc. ( μ g/$cm^3$) in Serum | | | | |
|---|---|---|---|---|---|
| | Hours: 1 | 2 | 3 | 5 | 7 |
| 1 | 0.74 | 1.04 | 1.79 | 1.66 | 0.97 |
| 2 | 0.16 | 0.39 | 0.48 | 0.54 | 0.43 |
| 3 | 0.57 | 1.02 | 1.32 | 1.68 | 1.33 |
| 4 | 0.35 | 0.57 | 1.02 | 1.03 | 0.74 |
| 5 | 0.14 | 0.26 | 0.41 | 0.51 | 0.38 |
| 6 | 0.16 | 0.35 | 0.37 | 0.35 | 0.22 |
| 7 | 0.14 | 0.26 | 0.46 | 0.49 | 0.36 |
| 8 | 0.62 | 0.94 | 1.26 | 1.25 | 0.94 |
| Mean | 0.36 | 0.60 | 0.89 | 0.94 | 0.67 |

TABLE 7 - Example 25

| Animal No | Levamisole conc. ( µ g/cm³) in Serum | | | | |
|---|---|---|---|---|---|
| | Hours: 1 | 2 | 3 | 5 | 7 |
| 1 | 0.33 | 0.74 | 0.74 | 0.74 | 0.76 |
| 2 | 0.12 | 0.33 | 0.45 | 0.54 | 0.50 |
| 3 | 0.41 | 0.78 | 0.94 | 0.91 | 0.85 |
| 4 | 0.33 | 0.54 | 0.72 | 0.70 | 0.41 |
| 5 | 0.10 | 0.21 | 0.27 | 0.41 | 0.30 |
| 6 | 0.31 | 0.54 | 0.62 | 0.70 | 0.48 |
| 7 | 0.31 | 0.62 | 0.91 | 1.01 | 0.85 |
| 8 | 0.21 | 0.43 | 0.56 | 0.62 | 0.37 |
| 9 | 0.21 | 0.45 | 0.52 | 0.58 | 0.41 |
| Mean | 0.26 | 0.52 | 0.64 | 0.69 | 0.55 |

TABLE 8 - Example 26

| Animal No | Levamisole conc. ( μ g/cm$^3$) in Serum | | | | |
|---|---|---|---|---|---|
| | Hours: 1 | 2 | 3 | 5 | 7 |
| 1 | 0.10 | 0.25 | 0.29 | 0.41 | 0.48 |
| 2 | 0.82 | 1.01 | 1.34 | 1.26 | 0.61 |
| 3 | 0.31 | 0.52 | 0.68 | 0.82 | 0.70 |
| 4 | 0.14 | 0.39 | 0.60 | 0.72 | 0.54 |
| 5 | 0.41 | 0.93 | 1.15 | 0.93 | 0.65 |
| 6 | 0.23 | 0.49 | 0.80 | 0.97 | 0.76 |
| 7 | 0.29 | 0.49 | 0.72 | 0.72 | 0.67 |
| 8 | 0.28 | 0.52 | 0.78 | 0.74 | 0.54 |
| 9 | 0.87 | 1.63 | 1.84 | 1.79 | 0.85 |
| 10 | 0.23 | 0.62 | 0.87 | 0.89 | 0.72 |
| Mean | 0.37 | 0.69 | 0.91 | 0.93 | 0.65 |

TABLE 9 - Example 27

| Animal No | Levamisole conc. ( μ g/cm³) in Serum | | | | |
|---|---|---|---|---|---|
| | Hours: 1 | 2 | 3 | 5 | 7 |
| 1 | 0.58 | 1.01 | 1.19 | 0.83 | 0.81 |
| 2 | 1.20 | 1.98 | 1.83 | 1.29 | 0.92 |
| 3 | 0.99 | 1.75 | 1.98 | 1.91 | 1.17 |
| 4 | 0.19 | 0.50 | 0.63 | 0.46 | 0.47 |
| 5 | 0.73 | 1.72 | 1.81 | 1.65 | 1.20 |
| Mean | 0.74 | 1.39 | 1.49 | 1.23 | 0.91 |

**TABLE 10** - Example 28

| Animal No | Levamisole conc. ( μ g/cm³) in Serum | | | | |
|---|---|---|---|---|---|
| Hours: | 1 | 2 | 3 | 5 | 7 |
| 1 | 0.53 | 0.62 | 0.77 | 0.79 | 0.55 |
| 2 | 1.10 | 1.21 | 1.33 | 1.14 | 0.84 |
| 3 | 0.39 | 0.53 | 0.78 | 0.78 | 0.75 |
| 4 | 0.30 | 0.41 | 0.68 | 0.90 | 0.98 |
| 5 | 0.21 | 0.23 | 0.26 | 0.28 | 0.26 |
| 6 | 1.05 | 1.16 | 1.83 | 1.41 | 1.66 |
| 7 | 0.69 | 0.95 | 1.37 | 1.76 | 1.70 |
| 8 | 0.30 | 0.38 | 0.51 | 0.48 | 0.40 |
| 9 | 0.72 | 0.94 | 1.68 | 1.80 | 1.33 |
| Mean | 0.59 | 0.71 | 0.96 | 1.04 | 0.94 |

TABLE 11 - Example 29

| Animal No | Levamisole conc. ( μ g/cm$^3$) in Serum | | | | |
|---|---|---|---|---|---|
| | Hours: 1 | 2 | 3 | 5 | 7 |
| 1 | 0.54 | 0.74 | 0.97 | 1.01 | 0.93 |
| 2 | 0.19 | 0.34 | 0.45 | 0.54 | 0.49 |
| 3 | 0.28 | 0.36 | 0.64 | 0.63 | 0.51 |
| 4 | 0.18 | 0.24 | 0.31 | 0.25 | 0.18 |
| 5 | 0.16 | 0.34 | 0.41 | 0.44 | 0.64 |
| 6 | 0.11 | 0.18 | 0.22 | 0.31 | 0.35 |
| 7 | 0.06 | 0.11 | 0.19 | 0.11 | 0.12 |
| 8 | 0.30 | 0.55 | 0.69 | 0.92 | 1.07 |
| 9 | 0.49 | 0.74 | 1.06 | 1.15 | 1.08 |
| 10 | 0.38 | 0.58 | 0.61 | 0.68 | 0.46 |
| Mean | 0.27 | 0.42 | 0.56 | 0.60 | 0.58 |

TABLE 12 - Example 30

| Animal No | Levamisole conc. ( µ g/cm$^3$) in Serum | | | | |
|---|---|---|---|---|---|
| | Hours: 1 | 2 | 3 | 5 | 7 |
| 11 | 0.13 | 0.21 | 0.31 | 0.39 | 0.46 |
| 12 | 0.13 | 0.23 | 0.27 | 0.38 | 0.35 |
| 13 | 0.13 | 0.15 | 0.17 | 0.12 | 0.10 |
| 14 | 0.17 | 0.35 | 0.45 | 0.56 | 0.58 |
| 15 | 0.15 | 0.20 | 0.24 | 0.18 | 0.33 |
| 16 | 0.31 | 0.58 | 0.67 | 0.57 | 0.62 |
| 17 | 0.05 | 0.19 | 0.14 | 0.20 | 0.18 |
| 18 | 0.17 | 0.34 | 0.42 | 0.58 | 0.40 |
| 19 | 0.26 | 0.45 | 0.58 | 0.59 | 0.69 |
| 20 | 0.17 | 0.31 | 0.43 | 0.40 | 0.40 |
| Mean | 0.17 | 0.20 | 0.37 | 0.40 | 0.41 |

TABLE 13 - Example 31

| Animal No | Levamisole conc. ( μ g/cm$^3$) in Serum | | | | |
|---|---|---|---|---|---|
| | Hours: 1 | 2 | 3 | 5 | 7 |
| 21 | 0.16 | 0.35 | 0.49 | 0.54 | 0.45 |
| 22 | 0.11 | 0.18 | 0.24 | 0.29 | 0.23 |
| 23 | 0.18 | 0.20 | 0.34 | 0.46 | 0.43 |
| 24 | 0.35 | 0.64 | 0.68 | 0.67 | 0.65 |
| 25 | 0.15 | 0.26 | 0.48 | 0.64 | 0.73 |
| 26 | 0.53 | 0.68 | 0.80 | 0.69 | 0.49 |
| 27 | 0.10 | 0.16 | 0.21 | 0.27 | 0.23 |
| 28 | 0.11 | 0.16 | 0.25 | 0.27 | 0.31 |
| 29 | 0.28 | 0.27 | 0.26 | 0.22 | 0.16 |
| 30 | 0.11 | 0.24 | 0.27 | 0.24 | 0.22 |
| Mean | 0.21 | 0.31 | 0.40 | 0.43 | 0.39 |

TABLE 14 - Example 32

| Animal No | Levamisole conc. ( μ g/cm$^3$) in Serum | | | | |
|---|---|---|---|---|---|
| | Hours: 1 | 2 | 3 | 5 | 7 |
| 31 | 0.27 | 0.57 | 0.76 | 0.85 | 0.62 |
| 32 | 0.23 | 0.35 | 0.49 | 0.59 | 0.64 |
| 33 | 0.19 | 0.35 | 0.39 | 0.30 | 0.25 |
| 34 | 0.14 | 0.21 | 0.30 | 0.38 | 0.34 |
| 35 | 0.50 | 0.77 | 0.87 | 0.83 | 0.65 |
| 36 | 0.29 | 0.47 | 0.54 | 0.69 | 0.48 |
| 37 | 0.15 | 0.11 | 0.13 | 0.14 | 0.12 |
| 38 | 0.10 | 0.23 | 0.38 | 0.42 | 0.35 |
| 39 | 0.10 | 0.15 | 0.16 | 0.14 | 0.14 |
| 40 | 0.42 | 0.56 | 0.73 | 0.72 | 0.64 |
| Mean | 0.24 | 0.38 | 0.48 | 0.51 | 0.42 |

TABLE 15 - Example 33

| Animal No | Levamisole conc. ( μ g/cm$^3$) in Serum | | | | |
|---|---|---|---|---|---|
| | Hours: 1 | 2 | 3 | 5 | 7 |
| 41 | 0.04 | 0.16 | 0.18 | 0.29 | 0.31 |
| 42 | 0.24 | 0.24 | 0.24 | 0.17 | 0.13 |
| 43 | 0.10 | 0.29 | 0.36 | 0.42 | 0.31 |
| 44 | 0.06 | 0.11 | 0.12 | 0.21 | 0.11 |
| 45 | 0.29 | 0.48 | 0.59 | 0.56 | 0.49 |
| 46 | 0.30 | 0.52 | 0.76 | 0.66 | 0.58 |
| 47 | 0.26 | 0.65 | 0.87 | 0.88 | 0.59 |
| 48 | 0.11 | 0.13 | 0.15 | 0.10 | 0.07 |
| 49 | 0.25 | 0.44 | 0.62 | 0.55 | 0.55 |
| 50 | 0.42 | 0.66 | 0.71 | 0.64 | 0.51 |
| Mean | 0.21 | 0.37 | 0.45 | 0.45 | 0.37 |

TABLE 16 - Example 34

| Animal No | Levamisole conc. ( μ g/cm³) in Serum | | | | |
|---|---|---|---|---|---|
| | Hours: 1 | 2 | 3 | 5 | 7 |
| 51 | 0.19 | 0.37 | 0.54 | 0.69 | 0.76 |
| 52 | 0.23 | 0.21 | 0.19 | 0.12 | 0.07 |
| 53 | 0.21 | 0.28 | 0.44 | 0.42 | 0.35 |
| 54 | 0.47 | 0.65 | 0.90 | 0.96 | 0.71 |
| 55 | 0.14 | 0.19 | 0.21 | 0.31 | 0.24 |
| 56 | 0.79 | 1.34 | 1.24 | 1.16 | 0.84 |
| 57 | 0.42 | 0.56 | 0.94 | 1.15 | 0.91 |
| 58 | 0.14 | 0.32 | 0.36 | 0.50 | 0.45 |
| 59 | 0.20 | 0.48 | 0.48 | 0.53 | 0.43 |
| 60 | 0.19 | 0.39 | 0.45 | 0.66 | 0.69 |
| Mean | 0.30 | 0.48 | 0.58 | 0.65 | 0.55 |

TABLE 17 - Example 35

| Animal No | Levamisole conc. ( μ g/cm³) in Serum | | | | |
|---|---|---|---|---|---|
| | Hours: 1 | 2 | 3 | 5 | 7 |
| 1 | 0.19 | 0.21 | 0.38 | 0.40 | 0.29 |
| 2 | 0.14 | 0.21 | 0.48 | 0.53 | 0.41 |
| 3 | 0.38 | 0.58 | 0.74 | 0.63 | 0.41 |
| 4 | 0.10 | 0.15 | 0.11 | 0.12 | 0.07 |
| 5 | 0.35 | 0.61 | 0.87 | 0.90 | 0.67 |
| 6 | 0.42 | 0.55 | 0.88 | 0.72 | 0.55 |
| 7 | 0.12 | 0.21 | 0.32 | 0.38 | 0.29 |
| 8 | 0.10 | 0.20 | 0.14 | 0.15 | 0.10 |
| Mean | 0.23 | 0.34 | 0.49 | 0.48 | 0.35 |

Example 36

This Example illustrates the efficacy of the compositions of the present invention in producing surprisingly high blood levels of levamisole in sheep in comparison to prior art compositions.

Ten freshly shorn Merino wethers were divided into two groups of five animals. Each of the animals in one group was treated with the compositon of Example 1 at a dose rate of 20 mg/kg following the procedure described for Examples 21 to 35. Each of the animals in the other group was treated with the following Comparative Example A prior art formulation at the same dose rate and following the same procedure.

Comparative Example A

| Component | Concentration (parts by weight) |
|---|---|
| Levamisole | 9.80 |
| 2-(n-Butoxy)ethanol | 56.39 |
| Ethylene Glycol Diacetate | 33.80 |
| Edicol Erythrosine | 0.01 |
| Density | 1.02 g/$cm^3$ |

The levamisole blood levels in each of the sheep from the two groups were measured following the procedure described for Examples 21 to 35 and the results are shown in Tables 18 and 19 below. No unacceptable adverse skin reactions were noted during or after the application of either composition.

The results clearly illustrate that the composition of the present invention provides a significantly higher (of the order of 4 to 8 fold higher) blood levamisole level in sheep than provided by the prior art composition. Moreover, the blood levamisole level provided by the composition of the present invention is well in excess of the desired level of 0.3 $\mu$ g/$cm^3$ which is generally recognized in the art to give effective control of helminth endoparasites, whereas the blood levamisole level provided by the prior art composition is below this desired level.

TABLE 18 - Example 36

| Animal No | Levamisole conc. ( μ g/cm³) in Serum | | | | |
|---|---|---|---|---|---|
| | Hours: 1 | 2 | 3 | 5 | 7 |
| 1 | 0.25 | 0.58 | 0.93 | 0.77 | 0.62 |
| 2 | 0.11 | 0.35 | 0.60 | 0.66 | 0.78 |
| 3 | 0.67 | 1.14 | 1.66 | 1.40 | 0.93 |
| 4 | 0.26 | 0.59 | 0.81 | 1.00 | 1.23 |
| 5 | 0.22 | 0.53 | 0.72 | 0.87 | 0.85 |
| Mean | 0.30 | 0.64 | 0.94 | 0.94 | 0.88 |

**TABLE 19 - Comparative Example A**

| Animal No | Levamisole conc. ( μ g/cm³) in Serum | | | | |
|---|---|---|---|---|---|
| | Hours: 1 | 2 | 3 | 5 | 7 |
| 6 | 0.06 | 0.07 | 0.11 | 0.11 | 0.12 |
| 7 | 0.04 | 0.11 | 1.19 | 0.25 | 0.33 |
| 8 | 0.00 | 0.05 | 0.07 | 0.07 | 0.06 |
| 9 | 0.06 | 0.15 | 0.16 | 0.25 | 0.28 |
| 10 | 0.06 | 0.22 | 0.35 | 0.35 | 0.44 |
| Mean | 0.04 | 0.12 | 0.18 | 0.21 | 0.25 |

Example 37

This Example illustrates the efficacy of the compositions of the present invention in producing surprisingly high blood levels of levamisole in cattle in comparison to prior art compositions.

Ten cattle, a mixture of Hereford and Angus calves, were divided into two groups of five animals. Each of the animals in one group was treated with the composition of Example 4 at a dose rate of 10 mg/kg, the composition being applied as a stripe along the backline of the animal. Each of the animals in the other group was treated with the following Comparative Example B prior art formulation at the same dose rate and following the same procedure.

Comparative Example B

| Component | Concentration (parts by weight) |
|---|---|
| Levamisole | 19.23 |
| 2-(n-Butoxy)ethanol | 37.34 |
| Ethylene glycol diacetate | 43.38 |
| 2,6-Di(t-Butyl)-4-methylphenol | 0.05 |
| Density | 1.04 $g/cm^3$ |

The levamisole blood levels of each of the animals from the two groups were measured following the procedure described for Examples 21 to 35 and the results are shown in Tables 20 and 21 below. No unacceptable adverse skin reactions were noted during or after the application of either composition.

The results clearly illustrate that the composition of the present invention provides a significantly higher (of the order of 2 to 3 fold higher during the first three hours after treatment) blood levamisole level in cattle than that provided by the prior art composition. Moreover, the blood levamisole level provided by the composition of the present invention is clearly well in the excess of the desired level of 0.3 $\mu$ $g/cm^3$ which is generally recognized in the art to give control of helminth endoparasites, whereas the blood levamisole level provided by the prior art composition approaches but does not clearly exceed this desired level.

TABLE 20 - Example 37

| Animal No | Levamisole conc. ( μ g/$cm^3$) in Serum | | | | |
| --- | --- | --- | --- | --- | --- |
| | Hours: 1 | 2 | 3 | 5 | 7 |
| 1 | 0.21 | 0.48 | 0.55 | 0.41 | 0.13 |
| 2 | 0.39 | 0.66 | 0.76 | 0.41 | 0.26 |
| 3 | 0.59 | 0.73 | 0.52 | 0.26 | 0.15 |
| 4 | 0.37 | 0.43 | 0.31 | 0.20 | 0.15 |
| 5 | 0.39 | 0.64 | 0.68 | 0.39 | 0.29 |
| Mean | 0.39 | 0.59 | 0.56 | 0.33 | 0.20 |

TABLE 21 - Comparative Example B

| Animal No | Levamisole conc. ( μ g/cm³) in Serum | | | | |
|---|---|---|---|---|---|
| Hours: | 1 | 2 | 3 | 5 | 7 |
| 6 | 0.18 | 0.41 | 0.44 | 0.33 | 0.24 |
| 7 | 0.11 | 0.16 | 0.17 | 0.13 | 0.11 |
| 8 | 0.20 | 0.43 | 0.48 | 0.29 | 0.20 |
| 9 | 0.05 | 0.25 | 0.35 | 0.44 | 0.33 |
| 10 | 0.12 | 0.23 | 0.26 | 0.20 | 0.33 |
| Mean | 0.13 | 0.30 | 0.34 | 0.28 | 0.24 |

Example 38

This Example illustrates the efficacy of topically applied compositions of the present invention in controlling helminth infestations in sheep in comparison to prior-art compositions administered by oral drenching.

Twenty-six Merino cross lambs (eleven months old with an average wool length of 3 to 4 cm) were infected with a variety of abomasal and small intestine parasites. These parasites were allowed to reach maturity in the animals over a period of twenty-one days and the animals were divided into three treatment groups. Eight animals (Animal Nos 1 to 8) were used as negative controls and received no treatment (Control Example). Nine animals (Animal Nos 9 to 17) were used as positive controls and were dosed orally with a commercial oral drench containing levamisole hydrochloride, at a levamisole hydrochloride dose rate of 7.5 mg/kg (Comparative Example C). The remaining nine animals (Animal Nos 18 to 26) were treated by topical application of the composition of Example 8, as a spot at the mid-point of the backline of each animal, and at a levamisole dose rate of 20 mg/kg.

Each of the animals was sacrificed seven days after treatment and the worms in the abomasum and small intestine were counted. The results are shown in Tables 22 to 23 and clearly demonstrate that the compositions of the present invention when topically applied are equally effective in eradicating endoparasites as prior-art commercial compositions applied by oral drenching.

TABLE 22 - Control Example

| Animal No | Post Mortem Worm count | | | | |
|---|---|---|---|---|---|
| | Abomasum | | | Small Intestine | |
| | HS | OS | TA | TS | NS |
| 1 | 300 | 1200 | 3900 | 9800 | 100 |
| 2 | 0 | 800 | 3300 | 9200 | 0 |
| 3 | 1540 | 4200 | 4400 | 8400 | 0 |
| 4 | 320 | 3100 | 3800 | 7100 | 0 |
| 5 | 2740 | 8400 | 1600 | 8600 | 0 |
| 6 | 1800 | 3600 | 3500 | 6700 | 0 |
| 7 | 1100 | 8400 | 4600 | 10400 | 100 |
| 8 | 0 | 1600 | 4100 | 5700 | 0 |
| Total | 7800 | 31300 | 29200 | 65900 | 200 |
| Mean | 975 | 3913 | 3650 | 8238 | 25 |

TABLE 23 - Comparative Example C

| Animal No | Post Mortem Worm count | | | | |
| --- | --- | --- | --- | --- | --- |
| | Abomasum | | | Small Intestine | |
| | HS | OS | TA | TS | NS |
| 9 | 0 | 110 | 50 | 10 | 0 |
| 10 | 0 | 60 | 0 | 10 | 0 |
| 11 | 0 | 0 | 0 | 0 | 0 |
| 12 | 0 | 10 | 90 | 0 | 0 |
| 13 | 0 | 10 | 0 | 0 | 0 |
| 14 | 0 | 10 | 20 | 0 | 0 |
| 15 | 0 | 10 | 490 | 20 | 0 |
| 16 | 0 | 70 | 230 | 10 | 0 |
| 17 | 0 | 180 | 130 | 0 | 0 |
| Total | 0 | 460 | 1010 | 50 | 0 |
| Mean | 0 | 51 | 112 | 6 | 0 |
| Efficacy | 100% | 98.7% | 97.0% | 99.9% | 100% |

TABLE 24 - Example 38

| Animal No | Post Mortem Worm count | | | | |
|---|---|---|---|---|---|
| | Abomasum | | | Small Intestine | |
| | HS | OS | TA | TS | NS |
| 18 | 0 | 60 | 20 | 30 | 0 |
| 19 | 0 | 0 | 0 | 0 | 0 |
| 20 | 0 | 0 | 0 | 10 | 0 |
| 21 | 0 | 80 | 190 | 60 | 0 |
| 22 | 0 | 540 | 250 | 240 | 0 |
| 23 | 0 | 10 | 90 | 0 | 0 |
| 24 | 0 | 100 | 180 | 10 | 0 |
| 25 | 0 | 230 | 370 | 50 | 0 |
| 26 | 0 | 40 | 40 | 0 | 0 |
| Total | 0 | 1060 | 1140 | 400 | 0 |
| Mean | 0 | 118 | 127 | 44 | 0 |
| Efficacy | 100% | 97.0% | 96.5% | 99.5% | 100% |

Code for TABLES 22 to 24

| | | |
|---|---|---|
| HS | - | *Haemonchus spp.* |
| OS | - | *Ostertagia spp.* |
| TA | - | *Trichostrongylus axei* |
| TS | - | *Trichostrongylus spp.* |
| NS | - | *Nematodirus spp.* |

Examples 39 and 40

These Examples illustrate the markedly improved storage stability of compositions of the present invention in comparison to prior art compositions comprising a hydroxylic solvent and an alkanoic acid.

The compositions detailed in Table 25 were prepared following essentially the same procedure as that described for Examples 1 to 20.

TABLE 25

| Component | Component Concentration (parts by weight) | | |
|---|---|---|---|
| | Example No 39 | Example No 40 | Comparative Example D |
| Levamisole | 9.80 | 9.80 | 9.80 |
| 2-(n-Butoxy)ethyl acetate | 53.41 | 56.97 | - |
| 2-(n-Butoxy)ethanol | - | - | 53.41 |
| Ethylene glycol diacetate | 33.18 | 33.18 | 33.18 |
| Propionic Acid | 3.56 | - | 3.56 |
| 2,6-Di(t-Butyl)-4-methylphenol | 0.05 | 0.05 | 0.05 |

To simulate storage at 20 °C over a period of approximately two years, samples of the compositions of Examples No 39 and 40 and Comparative Example D were placed in sealed glass containers and held at

80 °C over a period of eleven days. Reference samples of each formulation were stored in sealed glass containers at a temperature of -20 °C for the same period.

After storage each formulation was analysed for levamisole content using vapour phase chromatography (3% OV 25 at 250 °C using dipentyl phthalate as internal standard). The results are shown in Table 26 below where the levamisole content is expressed as the percentage levamisole remaining in the test sample in comparison to the reference sample.

TABLE 26

| Compositions | Levamisole Content (%) |
|---|---|
| Example 39 | 91.5 |
| Example 40 | 91.5 |
| Comparative Example D | 10.0 |

Example 41

This Example illustrates the freedom from unacceptable adverse skin reaction observed with compositions of the present invention in comparison to prior-art compositions comprising a hydroxylic solvent.

A group of eight Merino sheep were selected, two were treated with a composition comprising 10% w/v levamisole in 2-(n-butoxy)ethanol (Comparative Example E) and six were treated with the composition of Example No 7, following the same procedure as that described for Examples 21 to 35. The animals were examined for signs of skin irritancy 14 days, and then again 28 days, after treatment.

The results are shown in Table 27 and clearly demonstrate that when compositions of the present invention are topically applied to sensitive animals such as sheep, no unacceptable adverse skin reaction occurs. Whereas in contrast, topical application of prior-art compositions comprising a hydroxylic solvent as carrier results in unacceptable adverse skin reaction which in turn reduces the quality and value of the animals fleece.

TABLE 27

| Composition | Sheep No | Skin Irritancy | |
|---|---|---|---|
| | | At 14 days | At 28 days |
| Example 7 | 1 | None | None |
| " " | 2 | None | None |
| " " | 3 | None | None |
| " " | 4 | None | None |
| " " | 5 | None | None |
| " " | 6 | None | None |
| Comparative Example E | 7 | Hard scab in fleece | Hard scab in fleece and break in wool |
| Comparative Example E | 8 | Hard scab in fleece | Hard scab in fleece and break in wool |

## Claims

1. A composition for topical application to animals to control endoparasites which composition comprises an endoparasiticide and a carrier comprising at least 50% of at least one $C_2$ to $C_7$ saturated aliphatic carboxylic ester of an ethylene glycol or propylene glycol mono-($C_1$ to $C_6$ alkyl) ether, and optionally up to 50% of an additional organic solvent selected from di($C_2$ to $C_6$ alkyl) esters of $C_2$ to $C_6$ dicarboxylic acids, di($C_2$ to $C_6$ carboxyl) esters of ethylene glycol, diethylene glycol, triethylene glycol, propylene glycol and butane-1,4-diol, tri($C_2$ to $C_6$ carboxyl) esters of glycerol, and the products of reaction of one mole of a $C_1$ to $C_6$ alcohol with from one to three moles of an alkylene oxide selected from ethylene oxide, propylene oxide and mixtures thereof.

2. A composition according to claim 1 wherein said saturated aliphatic carboxylic ester is a compound of formula I:

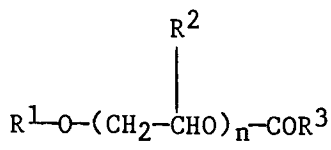

I

wherein:

- $R^1$ is selected from $C_1$ to $C_6$ alkyl;
- $R^2$ is selected from hydrogen and methyl;
- $R^3$ is selected from $C_1$ to $C_6$ alkyl; and
- n is an integer selected from 1 to 3.

**3.** A composition according to any one of claims 1 and 2 wherein said saturated aliphatic carboxylic ester is selected from the $C_2$ to $C_4$ alkanoate esters of 2-(n-butoxy)ethanol, 2-[2-(n-butoxy)ethoxy]ethanol, 1-(n-butoxy)propan-2-ol, 2-(n-propoxy)-ethanol, 2-[2-(n-propoxy)ethoxy]ethanol, 2-(2-ethoxy-ethoxy)ethanol and 1-ethoxypropanol.

**4.** A composition according to any one of claims 1 to 3 inclusive wherein said ester is 2-(n-butoxy)ethyl acetate.

**5.** A composition according to any one of claims 1 to 4 inclusive wherein the optional additional organic solvent is selected from: di($C_1$ to $C_6$ alkyl) esters of $C_2$ to $C_6$ dicarboxylic acids; and the di($C_2$ to $C_6$ carboxyl) esters of ethylene glycol, diethylene glycol, triethylene glycol, propylene glycol and butan-1,4-diol.

**6.** A composition according to any one of claims 1 to 5 inclusive wherein the optional additional organic solvent is selected from ethylene glycol diacetate, diethylene glycol diacetate and butan-1,4-diol diacetate.

**7.** A composition according to any one of claims 1 to 7 inclusive wherein said endoparasiticide is selected from: tetramisole, levamisole, the avermectins, triclabendazole, and rafoxanide.

**8.** A composition according to any of claims 1 to 7 inclusive wherein said composition additionally comprises one or more aliphatic carboxylic acids.

**9.** A composition according to any one of claims 1 to 8 inclusive wherein said composition additionally comprises an ectoparasiticide selected from the synthetic pyrethroids permethrin, cypermethrin, deltamethrin, phenothrin, cyphenothrin, flumethrin, cyfluthrin and cyhalothrin.

## Revendications

**1.** Composition pour application topique à des animaux, destinée à contrôler les endoparasites, laquelle composition comprend un endoparasiticide et un support comprenant au moins 50% d'au moins un ester carboxylique aliphatique saturé $C_2$ à $C_7$ d'un éther mono -(alkylique $C_1$ à $C_6$) d'éthylèneglycol ou de propylèneglycol, et optionnellement jusqu'à 50% d'un solvant organique supplémentaire choisi parmi les esters di -(alkyliques $C_2$ à $C_6$) d'acides dicarboxyliques $C_2$ à $C_6$, les esters di -(carboxyliques $C_2$ à $C_6$) d'éthylèneglycol, de diéthylèneglycol, de triéthylèneglycol, de propylèneglycol et du butanediol -1,4, les esters tri (carboxyliques $C_2$ à $C_6$) de glycerol, et les produits de réaction d'une mole d'un alcool $C_1$ à $C_6$ avec une à trois modes d'un oxyde d'alkylène choisi parmi l'oxyde d'éthylène, l'oxyde de prolylène et leurs mélanges.

**2.** Composition selon la revendication 1, dans laquelle ledit ester carboxylique aliphatique saturé est un composé de formule I:

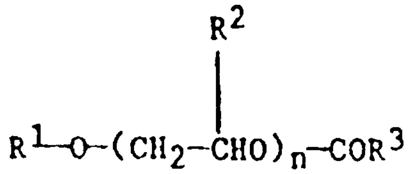

I

où:

- $R^1$ est choisi parmi les alkyles $C_1$ à $C_6$ ;
- $R^2$ est choisi parmi l'hydrogène et le méthyle ;
- $R^3$ est choisi parmi les alkyles $C_1$ à $C_6$ ; et
- n est un entier choisi de 1 à 3.

**3.** Composition selon l'une quelconque des revendications 1 et 2, dans laquelle ledit ester carboxylique aliphatique saturé est choisi parmi les esters alcanoates $C_2$ à $C_4$ de (n -butoxy) -2 éthanol, de [(n -butoxy) -2 éthoxy] -2 éthanol, de (n -butoxy) - 1 propanol -2, de (n -propoxy) -2 éthanol, de [(n -propoxy) -2 éthoxy] -2 éthanol, d'(éthoxy -2 éthoxy) -2 éthanol et d'éthoxy -1 propanol.

**4.** Composition selon l'une quelconque des revendications 1 à 3 inclusivement, dans laquelle ledit ester est l'acétate de (n -butoxy) -2 éthyle.

**5.** Composition selon l'une quelconque des revendications 1 à 4 inclusivement, dans laquelle le solvant organique supplémentaire optionnel est choisi parmi : les esters di (alkyliques $C_1$ à $C_6$) d'acides dicarboxyliques $C_2$ à $C_6$ et les esters di (carboxyliques $C_2$ à $C_6$) d'éthylèneglycol, de diéthylèneglycol, de triéthylèneglycol, de propylèneglycol et de butanediol -1,4.

**6.** Composition selon l'une quelconque des revendications 1 à 5 inclusivement, dans laquelle le solvant organique supplémentaire optionnel est choisi parmi le diacétate d'éthylèneglycol, le diacétate de diéthylèneglycol, et le diacétate de butanediol -1,4.

**7.** Composition selon l'une quelconque des revendications 1 à 6 inclusivement, dans laquelle ledit endoparasiticide est choisi parmi : le tétramisole, le levamisole, les avermectines, le triclabendazole, et le rafoxanide.

**8.** Composition selon l'une quelconque des revendications 1 à 7 inclusivement, comprenant en outre un ou plusieurs acides carboxyliques aliphatiques.

**9.** Composition selon l'une quelconque des revendications 1 à 8 inclusivement, comprenant en outre un ectoparasiticide choisi parmi les pyrethroïdes synthétiques suivants : la perméthrine, la cyperméthrine, la deltaméthrine, la phénothrine, la cyphenothrine, la fluméthrine, la cyfluthrine, et la cyhalothrine.

## Patentansprüche

**1.** Zusammensetzung zur topischen Anwendung bei Tieren zur Bekämpfung von Endoparasiten, umfassend ein Endoparasitizid und einen Träger, enthaltend mindestens 50 % von mindestens einem $C_2$- bis $C_7$-gesättigten aliphatischen Carbonsäureester eines Ethylenglykol- oder Propylenglykol-mono-($C_1$- bis $C_6$-alkyl)-ethers und gegebenenfalls bis zu 50 % eines zusätzlichen organischen Lösungsmittels, das unter Di-($C_2$- bis $C_6$-alkyl)-estern von $C_2$- bis $C_6$-Dicarbonsäureestern, Di-($C_2$- bis $C_6$-carboxyl)-estern von Ethylenglykol, Diethylenglykol, Triethylenglykol, Propylenglykol und Butan-1,4-diol, Tri-($C_2$- bis $C_6$-carboxyl)-estern von Glycerin und den Reaktionsprodukten von 1 Mol eines $C_1$- bis $C_6$-Alkohols mit 1 bis 3 Mol eines unter Ethylenoxid, Propylenoxid und Gemischen davon ausgewählten Alkylenoxids ausgewählt ist.

**2.** Zusammensetzung nach Anspruch 1, wobei es sich beim gesättigten aliphatischen Carbonsäureester um eine Verbindung der Formel I handelt:

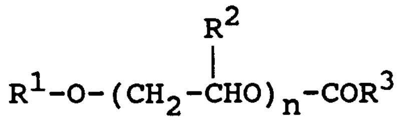

I

worin

| | |
|---|---|
| $R^1$ | die Bedeutung $C_1$- bis $C_6$-Alkyl hat; |
| $R^2$ | unter Wasserstoff und Methyl ausgewählt ist; |
| $R^3$ | unter $C_1$- bis $C_6$-Alkyl ausgewählt ist; und |
| n | eine ganze Zahl von 1 bis 3 bedeutet. |

3. Zusammensetzung nach einem der Ansprüche 1 und 2, wobei der gesättigte aliphatische Carbonsäureester unter $C_2$- bis $C_4$-Alkanoatestern von 2-(n-Butoxy)-ethanol, 2-[2-(n-Butoxy)-ethoxy]-ethanol, 1-(n-Butoxy)-propan-2-ol, 2-(n-Propoxy)-ethanol, 2-[2-(n-Propoxy)-ethoxy]-ethanol, 2-(2-Ethoxy-ethoxy)-ethanol und 1-Ethoxypropanol ausgewählt ist.

4. Zusammensetzung nach einem Ansprüche 1 bis 3, wobei es sich bei dem Ester um 2-(n-Butoxy)-ethylacetat handelt.

5. Zusammensetzung nach einem der Ansprüche 1 bis 4, wobei das gegebenenfalls vorhandene zusätzliche organische Lösungsmittel ausgewählt ist unter Di-($C_1$- bis $C_6$-alkyl)-estern von $C_2$- bis $C_6$-Dicarbonsäuren und den Di($C_2$- bis $C_6$-carboxyl)-estern von Ethylenglykol, Diethylenglykol, Triethylenglykol, Propylenglykol und Butan-1,4-diol.

6. Zusammensetzung nach einem der Ansprüche 1 bis 5, wobei das gegebenenfalls vorhandene zusätzliche organische Lösungsmittel ausgewählt ist unter Ethylenglykoldiacetat, Diethylenglykoldiacetat und Butan-1,4-dioldiacetat.

7. Zusammensetzung nach einem der Ansprüche 1 bis 7, wobei das Endoparasitizid ausgewählt ist unter Tetramisol, Levamisol, den Avermectinen, Triclabendazol und Rafoxanid.

8. Zusammensetzung nach einem der Ansprüche 1 bis 7, wobei die Zusammensetzung zusätzlich eine oder mehrere aliphatische Carbonsäuren enthält.

9. Zusammensetzung nach einem der Ansprüche 1 bis 8, wobei die Zusammensetzung zusätzlich ein Ektoparasitizid enthält, das ausgewählt ist unter den synthetischen Pyrethroiden Permethrin, Cypermethrin, Deltamethrin, Phenothrin, Cyphenothrin, Flumethrin, Cyfluthrin und Cyhalothrin.